(19) Europäisches Patentamt
European Patent Office
Office européen des brevets

(11) EP 1 021 161 B1

(12) FASCICULE DE BREVET EUROPEEN

(45) Date de publication et mention de la délivrance du brevet:
**08.01.2003 Bulletin 2003/02**

(21) Numéro de dépôt: **98946538.0**

(22) Date de dépôt: **01.10.1998**

(51) Int Cl.[7]: **A61K 7/48**, A61K 7/06

(86) Numéro de dépôt international:
**PCT/FR98/02098**

(87) Numéro de publication internationale:
**WO 99/016415 (08.04.1999 Gazette 1999/14)**

# (54) UTILISATION DE L'ACIDE ELLAGIQUE ET DE SES DERIVES EN COSMETIQUE ET EN DERMATOLOGIE

VERWENDUNG VON ELLAGSÄURE UND IHREN DERIVATEN IN DER KOSMETIK UND DER DERMATOLOGIE

USE OF ELLAGIC ACID AND ITS DERIVATIVES IN COSMETICS AND DERMATOLOGY

(84) Etats contractants désignés:
**AT BE CH CY DE DK ES FI FR GB GR IE IT LI LU MC NL PT SE**

(30) Priorité: **01.10.1997 FR 9712227**

(43) Date de publication de la demande:
**26.07.2000 Bulletin 2000/30**

(73) Titulaire: **LVMH RECHERCHE**
**75008 Paris (FR)**

(72) Inventeurs:
- **BONTE, Frédéric**
**F-45100 Orléans (FR)**
- **SAUNOIS, Alex**
**F-45240 Menestreau en Vilette (FR)**

(74) Mandataire: **Portal, Gérard et al**
**Cabinet Beau de Loménie**
**158, rue de l'Université**
**75340 Paris Cédex 07 (FR)**

(56) Documents cités:
**EP-A- 0 283 349**
**EP-A- 0 496 173**
**FR-A- 2 366 836**
**EP-A- 0 294 808**
**WO-A-95/21018**
**US-A- 5 141 741**

- **DATABASE WPI Week 9043 Derwent Publications Ltd., London, GB; AN 90-324222 XP002067703 & JP 02 231423 A (LION CORP)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 1 021 161 B1

## Description

La présente invention concerne essentiellement une nouvelle utilisation de l'acide ellagique, de ses sels, de ses complexes métalliques, de ses dérivés mono- ou poly-éthers, mono- ou poly-acylés dans le domaine de la cosmétique ou de la pharmacie, notamment en dermatologie.

L'acide ellagique, également dénommé : 2,3,7,8-tétrahydroxy(1)-benzopyrano(5,4,3-cde)(1)benzopyran-5,10 dione est une molécule bien connue appartenant au groupe des polyphénols et présente dans le règne végétal. On pourra se reporter à la publication du Merck Index 12ème édition (1996), n°3588.

On connaît par le document FR-A-1 478 523 un procédé de purification de l'acide ellagique ainsi que les acides ellagiques purifiés obtenus par un tel procédé.

L'acide ellagique présente la formule chimique suivante :

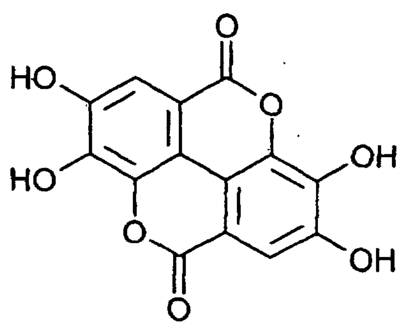

qui comporte quatre cycles accolés.

L'acide ellagique est disponible dans le commerce notamment auprès de la société Extra Synthèse, France.

Le document EP-A-0 496 173 décrit des extraits de noix d'Alep contenant de l'acide ellagique en combinaison avec de l'acide gallique et des tanins hydrolysables ainsi qu'une application en cosmétique comme filtre protecteur des ultra-violets B et pour un rôle préventif contre les effets nocifs des radicaux libres, responsables du vieillissement cutané.

Par ailleurs, on sait que le collagène de type VII, ci-après désigné par collagène VII, est le constituant prédominant des fibrilles d'ancrage, associées à la membrane basale, reliant l'épiderme au derme. Il est synthétisé par les kératinocytes de la couche basale de l'épiderme, et, en quantité moindre, par les fibroblastes du derme comme décrit par R. Burgeson dans une publication intitulée : "Type VII collagen, anchoring fibrils, and epidermolysis bullosa", J.Invest.Dermatol, (1993) 101,252-255. On pourra également se reporter à la publication de A. Koenig et al., dans J. Invest. Dermatol. (1992) 99 808-812. A ce propos, on notera que, selon des études récentes, des applications topiques d'acide rétinoique augmentaient le nombre de fibrilles d'ancrage sur des peaux ayant subi un vieillissement actinique (Woodley D.T. et al., J. Amer. Med. Assoc. (1990) 263, 3057-9). Or, l'acide rétinoïque, ou trétinoïne, est reconnu comme étant l'un des agents anti-rides les plus performants (L.H. Kligman, Cutis (1988) 41 (6) 419-20 ; J.J. Leyden et al., J. Am. Acad. Dermatol. (1989) 21 (3Pt 2) 638-44 ; J.H. Saurat, Horm. Res. (1995) 43 (1-3) 89-92)

D'après la publication de Y.Q. Chen, A.Mauviel, J.Ryynanen, S.Sollberg, J.Uitto ("Type VII collagen gene expression by human skin fibroblasts and keratinocytes in culture : Influence of donor age and cytokine responses" J. Invest.Dermatol, (1994) 102, 205-209), certaines manifestations du vieillissement cutané, telles qu'une fragilité cutanée accrue et une diminution des capacités de réparation de l'épiderme, pourraient être attribuables à une diminution de la synthèse du collagène VII chez les sujets âgés. On notera que l'expression "fragilité cutanée" recouvre en particulier l'apparition de cloques au niveau sub-basal.

Il a été décrit par M. Akiyama et al. dans J. Invest. Dermatol. (1995) 105 844-850, que le collagène, en particulier le collagène VII, jouait un rôle important au niveau du follicule pileux humain, notamment au niveau des membranes basales de la matrice (zone péripapillaire) et au niveau de la membrane basale du bulge (renflement de la partie haute du bulbe). Ces deux zones contiennent des cellules à fort potentiel mitotique, en particulier des kératinocytes donnant naissance à la tige pilaire.

Enfin, il est également connu que la cohésion derme-épiderme est primordiale pour que les populations basales des kératinocytes épidermiques aient un métabolisme optimal. Une bonne cohésion leur permettant, de ce fait et en particulier, d'assurer la formation d'une couche cornée de bonne qualité, élastique et bien formée avec une hydratation interne optimale qui respecte les fonctionnalités des couches cellulaires. Une bonne cohésion derme-épiderme participe ainsi à la formation et à l'entretien d'une peau au métabolisme équilibré en lui donnant notamment un bon aspect esthétique.

Le document brevet EP-A-0 496 173 présente l'acide éllagique comme agent actif antiradicalaire de l'extrait de Noix d'Alep, permettant de lutter contre l'altération des protéines de structure telles que le collagène et contre la désorganisation des membranes dans l'épiderme.

Dans le cadre de la présente invention, il a été découvert de manière surprenante que l'acide ellagique, ses sels, ses complexes métalliques, ses dérivés mono- ou poly-éthers. mono- ou poly-acylés permettaient d'augmenter significativement le taux de collagène VII dans un milieu où sont présents des kératinocytes humains normaux.

Cette constatation a conduit à la mise au point d'une nouvelle composition cosmétique, pharmaceutique, notamment dermatologique, plus particulièrement utile dans toutes les applications où l'on cherche à obtenir une augmentation du taux de collagène VII, en vue et en particulier. d'une part, de favoriser la cohésion entre le derme et l'épiderme. et d'autre part, au niveau des follicules pileux du cuir chevelu, de contribuer à la restauration ou au maintien de la fonctionnalité des cellules, en particulier des kératinocytes. Cette propriété s'est avérée particulièrement utile pour l'élaboration de compositions topiques, cosmétiques ou dermatologiques.

De telles compositions permettent, en particulier, de favoriser la cohésion entre le derme et l'épiderme chez les personnes présentant une peau atone ou relâchée. Elles peuvent également être utiles pour les soins capillaires pour améliorer l'état de la chevelure, pour favoriser la croissance de cheveux de bonne qualité ou pour ralentir ou retarder la chute des cheveux. Elles permettent aussi de traiter les pathologies s'accompagnant d'une déficience de la jonction denno-épidermique, telle que l'épidermolyse bulleuse.

Ainsi, selon un premier aspect, l'invention concerne l'utilisation de l'acide ellagique, de ses sels. de ses complexes métalliques, de ses dérivés mono- ou poly-éthers, mono- ou poly-acylés comme agent cosmétique pour améliorer la cohésion entre le derme et l'épiderme, ledit agent étant de préférence incorporé dans une composition cosmétique comprenant un véhicule cosmétiquement acceptable.

Avantageusement, l'amélioration de la cohésion entre le derme et l'épiderme est réalisée par le renforcement de la jonction dermo-épidermique.

Selon un deuxième aspect, la présente invention couvre aussi l'utilisation de l'acide ellagique, de ses sels, de ses complexes métalliques, de ses dérivés mono- ou poly-éthers, mono- ou poly-acylés comme agent cosmétique destiné à augmenter le taux de collagène VII, ledit agent étant de préférence incorporé dans une composition cosmétique comprenant un véhicule cosmétiquement acceptable.

Dans le cadre de l'invention, il a été en effet mis clairement en évidence de manière surprenante que l'acide ellagique, ses sels, ses complexes métalliques, ses dérivés mono- ou poly-éthers, mono- ou poly-acylés agissent en augmentant le taux de collagène VII.

Ainsi, les compositions de l'invention s'avèrent particulièrement utiles dans toutes les applications où l'on cherche à améliorer la cohésion entre le derme et l'épiderme.

Dans le cadre de l'invention, les sels de l'acide ellagique comprennent en particulier les sels métalliques, notamment alcalins ou alcalino-terreux, tels que le sodium et le calcium, les sels d'amines tels que les sels de méthylglutamine, de diéthanolamine, de triéthanolamine, de choline, de bis-triéthylamine, les sels d'acides aminés notamment les sels d'acides aminés basiques tels que l'arginine, la lysine et l'ornithine, les complexes métalliques comprennent en particulier des complexes métalliques avec le zinc et le cuivre et les dérivés mono- ou poly-acylés comprennent en particulier des groupes acyles, saturés ou insaturés, ayant de 2 à 22 atomes de carbone. De préférence, ces groupes acyles correspondent aux acides acétique, palmitique, oléique, linoléique, linolénique, arachidonique, stéarique, brassidique, érucique, béhénique et (all Z)-5,8,11,14,17-eicosapentaènoïque. Les dérivés mono- ou poly-éthers précités sont en particulier des dérivés alcoxy comprenant de 1 à 4 atomes de carbone, ou bien des dérivés de condensation de l'un ou de plusieurs groupes hydroxy de l'acide ellagique avec un sucre ou une chaîne de sucres. En particulier, il s'agit de l'acide 3-méthoxy ellagique ou des dérivés mono- ou poly-éthers avec les sucres tels que le glucose, l'arabinose, le rhamnose et le galactose.

Les dérivés éthers ou acylés précités peuvent être obtenus par des procédés d'éthérification ou d'acylation des polyphénols bien connus de l'homme de l'art. Certains peuvent également être obtenus par extraction à partir de plantes.

En outre, l'acide ellagique, ses sels, ses complexes métalliques, ses dérivés mono- ou poly-éthers, mono- ou polyacylés seront particulièrement destinés à obtenir un raffermissement de la peau, à prévenir ou à retarder l'apparition des signes du vieillissement cutané, à retarder l'apparition des rides ou à diminuer leur profondeur, à améliorer l'état de la chevelure.

L'invention vise ainsi en particulier des produits anti-rides, des produits destinés à combattre le vieillissement cutané et en particulier le vieillissement naturel cutané, et le relâchement de la peau, ou une lotion de soin capillaire, telle qu'une lotion "anti-chute" des cheveux.

L'invention permet par voie de conséquence de préparer des compositions cosmétiques particulièrement intéressantes pour lutter contre le vieillissement cutané, en particulier contre le vieillissement actinique de la peau, c'est-à-dire le vieillissement induit par les rayonnements, en particulier le rayonnement solaire, tout particulièrement le rayonnement solaire ultraviolet.

De façon générale, les compositions cosmétiques de l'invention s'avèrent particulièrement utiles en tant que produit raffermissant de la peau destiné, en particulier, à lutter contre les peaux relâchées ou atones.

Selon un troisième aspect, l'invention concerne également l'utilisation de l'acide ellagique, de ses sels, de ses complexes métalliques, de ses dérivés mono- ou poly-éthers, mono- ou polyacylés pour la préparation d'une composition pharmaceutique, notamment dermatologique, destinée à traiter les pathologies liées à une déficience de la cohésion entre le derme et l'épiderme, en particulier celles liées à un affaiblissement de la jonction dermo-épidermique, telle que l'épidermolyse bulleuse, ou destinée à traiter les manifestations ou les pathologies liées à une insuffisance du taux de collagène VII.

Les compositions cosmétiques ou pharmaceutiques, notamment dermatologiques de l'invention, contiendront avantageusement de 0,0001 % à 5 %, de préférence entre 0,01 et 1 % en poids de l'acide ellagique, de ses sels, de ses complexes métalliques, de ses dérivés mono- ou poly-éthers, mono- ou poly-acylés par rapport au poids total de la composition.

Les compositions de l'invention pourront comprendre, en outre avantageusement, au moins une substance favorisant la synthèse des constituants de la matrice extra-cellulaire de la peau, et/ou régulant la formation d'une couche cornée de bonne qualité.

A titre d'exemple de telles substances, on citera les vitamines, en particulier les vitamines du groupe A et C et leurs dérivés tels que les esters, les tocophérols, les xanthines, en particulier la caféine ou la théophylline, les rétinoïdes, en particulier la vitamine A acide, les extraits de Centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica. des éléments minéraux, de préférence le magnésium. le manganèse, le silicium et le zinc. ces éléments minéraux étant utilisés avantageusement sous forme de chlorure pour le magnésium et le manganèse. sous forme de silanol pour le silicium et sous forme d'aspartate pour le magnésium.

Par ailleurs. la composition selon l'invention peut contenir, en outre, au moins une substance choisie parmi le groupe consistant des alpha-hydroxy-acides aliphatiques en $C_3$-$C_{12}$, en particulier l'acide citrique, l'acide malique et l'acide lactique. les acides aminés. en particulier l'arginine, la citrulline et la thréonine, les céramides. les glycocéramides. les phospholipides. les agents amincissants, la forskoline. les extraits de Coleus. les extraits de Tephrosia, les agents anti-vergetures, en particulier les extraits de Marron d'Inde et l'escine, les agents protégeant ou améliorant la microcirculation, en particulier les biotlavonoïdes de Ginkgo Biloba. et les filtres solaires, en particulier les oxydes de titane, le méthoxy-cinnamate d'acyle (Parsol MCX®) et les filtres d'origine végétale.

Dans les compositions de l'invention plus particulièrement destinées au traitement et au soin des cheveux, l'acide ellagique, ses sels, ses complexes métalliques, ses dérivés mono- ou poly-éthers, mono- ou poly-acylés sera avantageusement associé à au moins un autre principe actif choisi parmi le groupe constitué par les agents anti-pelliculaires, tels que les extraits d'Arctium lappa, le chloroxylénol. le résorcinol ou la pyrithione de zinc, les agents anti-séborrhéiques tels qu'un inhibiteur de 5$\alpha$-réductase, en particulier un extrait de Pygeum africanum et les agents stimulant la microcirculation sanguine tels que la cépharanthine et le nicotinate de méthyle.

Diverses formes de formulations peuvent être réalisées. L'une des formes les plus utilisées est une forme topique adaptée pour être appliquée sur le tissu cutané. Ces formulations topiques appropriées incluent, sans limitation, des émulsions, des crèmes, des laits, des baumes, des gels, des lotions ainsi que des compositions de maquillage traitantes.

L'invention concerne également selon d'autres aspects un procédé de traitement cosmétique ou pharmaceutique, notamment dermatologique, selon lequel on cherche à obtenir une augmentation du taux de collagène VII.

L'invention concerne aussi en particulier un procédé de traitement cosmétique destiné à obtenir une amélioration de la cohésion entre le derme et l'épiderme, en particulier par un renforcement de la jonction dermo-épidermique, caractérisé en ce qu'il consiste à délivrer une quantité cosmétiquement efficace de l'acide ellagique, de ses sels, de ses complexes métalliques, de ses dérivés mono- ou poly-éthers, mono- ou poly-acylés, éventuellement contenu dans un excipient cosmétiquement acceptable.

Naturellement, des modes de réalisations. en particulier de ce procédé, résultent de la description précédente relativement aux compositions.

D'autres buts. caractéristiques et avantages de l'invention apparaîtront clairement à l'homme de l'art, à partir de la description explicative qui va suivre faite en référence à plusieurs exemples de réalisation donnés simplement à titre d'illustration et qui ne sauraient en aucun cas limiter la portée de l'invention. Les exemples font partie intégrante de l'invention. D'autre part, dans les exemples qui suivent, les pourcentages sont indiqués en poids, les températures sont en degrés celsius, la pression est la pression atmosphérique sauf indication contraire.

EXEMPLES DE L'INVENTION

Exemple 1

Mise en évidence de l'activité de l'acide ellagique sur l'augmentation du taux de collagène VII dans une culture de kératinocytes humains normaux.

Le test ci-dessous a été réalisé à partir d'acide ellagique disponible chez Extra Synthèse, France, sous forme de poudre ayant un point de fusion supérieur à 360°C.

Les tests ont été réalisés en aveugle sur des kératinocytes humains normaux.

1 - Protocole du test

a) Provenance des kératinocytes:

Les cultures de kératinocytes humains normaux (KHN) sont établies à partir d'un prélèvement chirurgical de peau saine. Dans la présente étude, les tests ont été réalisés sur deux souches cellulaires provenant d'un lifting respectivement d'une femme caucasienne de 49 ans, noté KHN 1124, et d'une femme caucasienne de 50 ans, noté KHN 1106.

b) Conditions de culture :

Les kératinocytes sont maintenus dans le milieu SFM (Serum Free Medium) complet (noté SFMc, GIBCO). Les cellules ont été sous-cultivées une ou deux fois à partir de la primoculture (soit un passage, noté P1 ou deux passages noté P2), avant leurs utilisations pour le test.

c) Conditions de traitement :

L'ensemencement des cellules est réalisé en plaque de culture à 96 puits à raison de 25 000 KHN par puits dans le milieu SFMc. Après 24 h d'incubation nécessaire à la bonne adhérence des cellules, le milieu est remplacé par un milieu SFMc dilué à 2 %, limitant la prolifération des kératinocytes pendant le test. La concentration testée d'acide ellagique est de 0,5 microgrammes par ml dans le milieu d'étude, obtenue extemporanément à partir d'une solution-mère d'acide ellagique dans le DMSO à 0,5 mg par ml. La concentration finale de DMSO dans le milieu d'étude est donc de 0.1 % $V/V_{final}$. Cette concentration n'est pas toxique pour les cellules. Le témoin est constitué du milieu d'étude SFMc dilué à 2 % ajouté de 0,1 % de DMSO en V/V. Six cultures sont réalisées respectivement pour le produit testé et pour l'essai témoin.

Les cellules sont mises au contact du milieu de traitement pendant 72 h.

Les surnageants d'incubation sont prélevés en vue du dosage du collagène VII. Un dosage des protéines est effectué sur le tapis cellulaire restant dans les puits (méthode BCA, SIGMA), dans le but de rapporter les quantités de collagène VII aux taux de protéines cellulaires.

Une plaque traitée parallèlement est utilisée pour le test de viabilité XTT en utilisant le kit XTT de Boehringer référence 146 50 15, pour mesurer l'activité déshydrogénase mitochondriale des cellules viables. Aucune baisse significative de la viabilité n'a été détectée par ce test ainsi que par l'observation miscrocospique des cellules à la concentration testée de 0,5 µg (microgrammes) par ml.

d) Dosage ELISA du collagène VII :

Le protocole de dosage du collagène VII par une méthode ELISA a été adapté à partir de celui utilisé pour le dosage du collagène I (M. DUMAS, C. CHAUDAGNE, F. BONTE, A. MEYBECK : "In vitro biosynthesis of type I and III collagens by human dermal fibroblasts from donors of increasing age", Mechanisms of Ageing and Development, 73 (1994) 179-187).

Les modifications suivantes ont été apportées :

- 1er anticorps : Anticorps monoclonal de souris anti-collagène type VII humain, isotype IgG (Life Technologies, Réf. 12073-011, Lot FB2b01).
- 2ème anticorps : Anticorps de chèvre anti-IgG totaux de souris, couplé à la phosphatase alcaline (Interchim, Réf. 115-056-062, Lot 26793).

L'activité phosphatase alcaline est révélée par le substrat para-nitro-phényle-phosphate (PNPP) clivé par l'enzyme en para-nitro-phénol, dont l'absorbance est mesurée à 405 nanomètres.

e) Expression des résultats et interprétation statistique :

Les résultats de l'augmentation du taux de collagène VII dans la culture de kératinocytes sont exprimés en Activité de l'acide ellagique, noté A, où A est exprimé en pourcentage et correspond à la formule suivante :

$$A = \left(\frac{DORCae - DORCks}{DORCks}\right) x\ 100$$

où:

- L'indice "ae" correspond aux valeurs liées à une culture de kératinocytes faite en présence d'acide ellagique.
- L'indice "ks" correspond aux valeurs liées à une culture de kératinocytes faite en l'absence d'acide ellagique, également nommée culture témoin.
- DOR correspond à la densité optique réduite :

$$DOR = (DO - DO_{Blanc})$$

- DO correspond à une densité optique mesurée suite au traitement d'un surnageant.
- $DO_{Blanc}$ correspond à la densité optique mesurée sur le milieu de culture.
- DORC correspond à une DOR corrigée pour correspondre à une densité optique correspondant à 100 µg de protéines cellulaires dosées sur le puits correspondant.

Les résultats obtenus sur les cultures traitées (n=6) et témoins (n=6) sont comparés par le test de Student non apparié, le seuil de significativité retenu étant p<0,05.

L'activité du produit selon l'invention, à savoir l'acide ellagique, sur le taux de collagène VII dans le milieu de culture a ainsi fait l'objet d'un essai sur les deux souches différentes de kératinocytes précédemment indiquées, référencées KHN 1124 (P2) et KHN 1106 (P1).

2) Résultats - Conclusion

Les résultats sont donnés dans le tableau 1 ci-dessous, à partir de la moyenne des mesures sur les différentes cultures :

TABLEAU 1

| ACTIVITE DE L'ACIDE ELLAGIQUE SUR LE TAUX DE COLLAGENE VII | | | | |
|---|---|---|---|---|
| Souches de kératinocytes | Concentration en acide ellagique (µg/ml) | DORC pour 72 h d'incubation | p (test t) par rapport à la culture témoin | A pour 72 h d'incubation |
| KHN 1124P2 (F 49 ans, lifting) | 0 | 0,773 +/- 0,023 | | |
| | 0,5 | 1,098 +/- 0,100 | 0,0003 | +42% |
| KHN 1106P1 (F 50 ans, lifting) | 0 | 0,950 +/- 0,180 | | |
| | 0,5 | 1,563 +/- 0,240 | 0,0007 | +64% |

Les résultats figurant au tableau 1 montrent une augmentation importante et significative du taux de collagène VII présent dans les milieux de culture, due à la présence de l'acide ellagique selon l'invention à une concentration de 0,5 µg/ml.

Le collagène VII étant notamment le constituant principal des fibrilles d'ancrage, il ressort clairement de ce test que l'acide ellagique ainsi que ses dérivés, selon l'invention, peuvent être avantageusement utilisés comme agent pour renforcer la jonction dermo-épidermique, et améliorer ainsi la cohésion entre le derme et l'épiderme. L'acide ellagique et ses dérivés, selon l'invention, peuvent donc être avantageusement utilisés dans des compositions cosmétiques "anti-rides", anti-vieillissement", "raffermissantes" ainsi que dans des compositions dermatologiques pour

lutter contre l'épidermolyse bulleuse.

Il est, par ailleurs, connu que le collagène VII joue un rôle important au niveau du follicule pileux humain, d'où il ressort compte tenu des résultats expérimentaux ci-dessus, un grand intérêt de l'acide ellagique et de ses dérivés, selon l'invention, pour le soin des cheveux, et, d'une manière générale, pour l'amélioration de l'état de la chevelure.

Exemple 2 de l'invention

Composition de soins cosmétiques raffermissants:

- Acide ellagique disponible dans le commerce chez Extra Synthèse, France : 0,01 g
- Extrait de Centella asiatica : 0,1 g
- Excipient émulsionné sous forme d'émulsion huile-dans-eau, parfumé et conservateur : QSP 100 g

Cette composition combat le relâchement de la peau, et restaure sa fermeté. Elle peut être avantageusement utilisée par cures de 3 semaines en applications sur les zones du corps à traiter où la peau est relâchée.

Exemple 3 de l'invention

Emulsion dite "anti-âge"

- Acétate d'acide ellagique : 0,02 g
- Palmitate de vitamine A : 0,01 g
- Excipient émulsionné fluide parfumé : QSP 100 g

Cette émulsion peut être utilisée sur les zones du corps à traiter, en particulier sur le visage, de préférence tous les soirs. Cette émulsion contribue à retarder l'apparition des signes du vieillissement de la peau tels que les rides ou le relâchement cutané. Après un traitement quotidien de 6 mois environ, la peau devient plus lisse, plus souple et plus ferme. Elle reprend son éclat.

Exemple 4 de l'invention

Emulsion cosmétique raffermissante et "anti-vieillissement".

Cette émulsion est préparée à partir des 3 phases A, B, C suivantes :

Phase A

- Emulgade SE® (1) : 6 g
- Alcool de cétyle : 2 g
- BHT : 0,02 g
- Isononyl isononanoate : 6 g
- Propylparabène : 0,02 g
- Cocoglycérides : 6 g
- Centella asiatica : 0,1 g

Phase B

- Méthylparabène : 0,2 g
- Eau distillée : 67,64 g

Phase C

(1) L'Emulgade SE® est une composition de la société HENKEL, mélange de : glyceryl stearate (et) ceteareth-20 (et) ceteareth-10 (et) cetearyl alcohol (et) cetyl palmitate.

- Acide ellagique : 0,01 g
- Butylène glycol : 12 g
- Palmitate de vitamine A : 0,01 g.

La phase C est d'abord préparée en faisant subir un traitement aux ultrasons au mélange d'acide ellagique et de butylène glycol, et ce, pendant 15 minutes. On ajoute ensuite le palmitate de vitamine A.

La phase grasse A et la phase aqueuse B sont chauffées ensemble à 85°C. A 85°C, on émulsionne la phase B sur la phase A par agitation mécanique. L'agitation est maintenue alors que l'on refroidit l'émulsion obtenue jusqu'à 45°C, température à laquelle l'agitation est maintenue pendant 45 minutes.

On rajoute la phase C à l'émulsion phase A - phase B qui est maintenue à 45°C et qui subit une agitation. Cette agitation est maintenue pendant 5 minutes après l'ajout de la phase C.

Cette émulsion peut être utilisée sur les zones du corps à traiter, en particulier sur le visage, de préférence tous les soirs. Cette émulsion contribue à retarder l'apparition des signes du vieillissement cutané, tels que les rides, réduire la profondeur de ces dernières ou lutter contre le relâchement cutané. Après un traitement quotidien de six mois environ, la peau devient plus lisse, plus souple et plus ferme.

<u>Exemple 5 de l'invention</u>

<u>Emulsion cosmétique raffermissante et "anti-vieillissement".</u>

Cette émulsion est préparée avec les composés suivants :

- Eau distillée : 67,53 g
- Butylène glycol : 12,00 g
- Emulgade SE® : 6,00 g
- Isononyl isononanoate : 6,00 g
- Cocoglycérides : 6,00 g
- Alcool de cétyle : 2,00 g
- Méthylparabène : 0,20 g
- Centella asiatica : 0,10 g
- Palmitate de vitamine A : 0,10 g
- Sel de sodium de l'acide ellagique : 0,03 g
- BHT : 0,02 g
- Propylparabène : 0,02 g

L'Emulgade SE® est une composition décrite dans l'exemple précédent.

Cette émulsion peut être utilisée sur les zones du corps à traiter, en particulier sur le visage, de préférence tous les soirs. Cette émulsion contribue à retarder l'apparition des signes du vieillissement cutané, tels que les rides, réduire la profondeur de ces dernières ou lutter contre le relâchement cutané. Après un traitement quotidien de six mois environ, la peau devient plus lisse, plus souple et plus ferme.

<u>Exemple 6 de l'invention</u>

<u>Préparation dermatologique gélifiée pour le traitement de l'épidermolyse bulleuse</u>

- Complexe métallique de zinc de l'acide ellagique : 0,01 g
- Butylène glycol : 10 g
- Ethanol absolu : 20 g
- Eau distillée : 54,99 g
- Carbomer (Gel d'Ultrez®-10 de chez GOODRICH) à 2 % : 15 g

On peut appliquer ce gel localement sur les zones à traiter en cas d'épidermolyse, trois fois par jour pendant au moins quinze jours.

Exemple 7 de l'invention

Lotion capillaire "anti-chute" et "anti-pelliculaire"

On prépare une lotion capillaire en utilisant 0,01 g de complexe de zinc de l'acide ellagique; 0,005 g de chloroxylènol et 0,01 g de cépharantine avec un excipient alcoolique parfumé en quantité suffisante pour 100 g.

La lotion est utilisée par application matin et soir sur le cuir chevelu, suivi d'un léger massage. Après 8 à 15 jours de traitement, la chute des cheveux est nettement ralentie et les démangeaisons disparaissent. Les cures conseillées sont de 30 jours, espacées de 2 à 4 mois selon l'importance du problème capillaire à traiter.

Exemple 8 de l'invention

Emulsion cosmétique raffermissante et "anti-vieillissement"

- Acide ellagique : 0,50 g
- Palmitate de vitamine A : 0,01 g
- Centella asiatica : 6,00 g
- Excipient avec conservateur QSP 100,00 g

Cette émulsion peut être utilisée sur les zones du corps à traiter, en particulier sur le visage, de préférence tous les soirs. Cette émulsion contribue à retarder l'apparition des signes du vieillissement cutané, tels que les rides, à réduire la profondeur de ces dernières ou à lutter contre le relâchement cutané. Après un traitement quotidien de six mois environ. la peau devient plus lisse, plus souple et plus ferme.

## Revendications

1. Utilisation cosmétique de l'acide ellagique, de ses sels, de ses complexes métalliques, de ses dérivés mono- ou poly-éthers, mono- ou poly-acylés, comme agent cosmétique pour améliorer la cohésion entre le derme et l'épiderme, ledit agent étant incorporé dans une composition cosmétique comprenant un véhicule cosmétiquement acceptable.

2. Utilisation cosmétique de l'acide ellagique, de ses sels, de ses complexes métalliques, de ses dérivés mono- ou poly-éthers, mono- ou poly-acylés, comme agent cosmétique destiné à stimuler la synthèse du collagène VII, par les kératinocytes ou les fibroblastes ledit agent étant incorporé dans une composition cosmétique comprenant un véhicule cosmétiquement acceptable.

3. Utilisation selon la revendication 1 ou 2, **caractérisée en ce que** ladite composition est destinée à obtenir un raffermissement de la peau, à prévenir ou à retarder l'apparition des signes du vieillissement cutané, à retarder l'apparition des rides ou à diminuer leur profondeur.

4. Utilisation selon la revendication 3, **caractérisée en ce que** le vieillissement cutané est le vieillissement actinique, dû en particulier à l'action du rayonnement solaire.

5. Utilisation selon la revendication 2, **caractérisée en ce que** ladite composition est une composition capillaire destinée à améliorer l'état de la chevelure.

6. Utilisation de l'acide ellagique, de ses sels, de ses complexes métalliques, de ses dérivés mono- ou poly-éthers, mono- ou poly-acylés pour la préparation d'une composition pharmaceutique, notamment dermatologique, destinée à traiter les pathologies liées à une déficience de la cohésion entre le derme et l'épiderme, en particulier celles liées à un affaiblissement de la jonction dermo-épidermique.

7. Utilisation de l'acide ellagique, de ses sels, de ses complexes métalliques, de ses dérivés mono- ou poly-éthers, mono- ou poly-acylés pour la préparation d'une composition pharmaceutique, notamment dermatologique, destinée à stimuler la synthèse du collagène VII par les kératinocytes ou les fibroblastes afin de traiter les manifestations ou les pathologies liées à une insuffisance du taux de collagène VII.

8. Utilisation selon la revendication 6. **caractérisée en ce que** ladite composition est destinée au traitement de l'épidermolyse bulleuse.

9. Utilisation selon l'une des revendications 1 à 8, **caractérisée en ce que** les sels de l'acide ellagique comprennent un sel métallique, en particulier alcalin ou alcalino-terreux tel que le sodium et le calcium, les sels d'amines tels que les sels de méthylglutamine. de diéthanolamine, de triéthanolamine, de choline, de bis-triéthylamine, les sels d'acides aminés notamment les sels d'acides aminés basiques tels que l'arginine, la lysine et l'omithine, les complexes métalliques de l'acide ellagique comprennent un complexe métallique avec le zinc et le cuivre, et les dérivés mono- ou poly-acylés comprennent des groupes acyles, saturés ou insaturés. ayant de 2 à 22 atomes de carbone, de préférence ces groupes acyles correspondent aux acides acétique. palmitique, oléique, linoléique. linolénique, arachidonique, stéarique. brassidique, érucique, béhénique et (all Z)-5,8,11,14,17-eicosapentaènoïque: les dérivés mono- ou poly-éthers précités sont des dérivés alcoxy comprenant de 1 à 4 atomes de carbone, ou bien des dérivés de condensation de l'un ou de plusieurs groupes hydroxy de l'acide ellagique avec un sucre ou une chaîne de sucres, en particulier l'acide 3-méthoxy ellagique ou ses dérivés mono- ou poly-éthers avec les sucres tels que le glucose, l'arabinose, le rhamnose et le galactose.

10. Utilisation selon l'une des revendications 1 à 9, **caractérisée en ce que** ladite composition contient de 0.0001 % à 5 % et de préférence entre 0,01 % et 1 % en poids de l'acide ellagique, de ses sels, de ses complexes métalliques, de ses dérivés mono- ou poly-éthers, mono- ou poly-acylés par rapport au poids total de la composition.

11. Utilisation selon l'une des revendications 1 à 10, **caractérisée en ce que** ladite composition contient en outre au moins une substance favorisant la synthèse des constituants de la matrice extra-cellulaire de la peau, et/ou régulant la formation d'une couche cornée de bonne qualité.

12. Utilisation selon la revendication 11, **caractérisée en ce que** ladite substance est choisie parmi le groupe constitué par les vitamines, en particulier les vitamines du groupe A et C et leurs dérivés tels que les esters, les tocophérols, les xanthines, en particulier la caféine ou la théophylline, les rétinoïdes, en particulier la vitamine A acide, les extraits de Centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica, des éléments minéraux.

13. Utilisation selon la revendication 12, **caractérisée en ce que** les éléments minéraux précités sont choisis parmi le magnésium, le manganèse, le silicium et le zinc, ces éléments minéraux étant avantageusement utilisés sous forme de chlorure pour le magnésium et le manganèse, sous forme de silanol pour le silicium et sous forme d'aspartate pour le magnésium.

14. Utilisation selon l'une des revendications 1 à 13, **caractérisée en ce que** ladite composition contient en outre au moins une substance choisie dans le groupe constitué par les alpha-hydroxy-acides aliphatiques en $C_3$-$C_{12}$, en particulier l'acide citrique, l'acide malique et l'acide lactique, les acides aminés, en particulier l'arginine, la citrulline et la thréonine, les céramides, les glycocéramides, les phospholipides, les agents amincissants, en particulier la forskoline, les extraits de Coleus, les extraits de Tephrosia, les agents anti-vergetures, en particulier les extraits de Marron d'Inde et l'escine, les agents protégeant ou améliorant la microcirculation, en particulier les bioflavonoïdes de Ginkgo Biloba, et les filtres solaires, en particulier les oxydes de titane, le méthoxy-cinnamate d'acyle et les filtres d'origine végétale.

15. Utilisation selon l'une des revendications 1 à 14, **caractérisée en ce que** ladite composition contient en outre au moins un autre principe actif choisi parmi le groupe constitué par les agents anti-pelliculaires, tels que les extraits d'Arctium lappa, le chloroxylénol, le résorcinol ou la pyrithione de zinc, les agents anti-séborrhéiques tels qu'un inhibiteur de 5$\alpha$-réductase, en particulier un extrait de Pygeum africanum, et les agents stimulant la microcirculation sanguine, tels que la cépharanthine et le nicotinate de méthyle.

16. Procédé de traitement cosmétique destiné à obtenir une amélioration de la cohésion entre le derme et l'épiderme, en particulier par un renforcement de la jonction dermo-épidermique, et/ou à stimuler la synthèse du collagène VII, par les kératinocytes ou les fibroblastes **caractérisé en ce qu'**il consiste à délivrer une quantité cosmétiquement efficace de l'acide ellagique, de ses sels, de ses complexes métalliques, de ses dérivés mono- ou poly-éthers, mono- ou poly-acylés, éventuellement contenus dans une composition comprenant un excipient cosmétiquement acceptable, en particulier sous une forme telle que définie à l'une des revendications 9 à 15.

## Claims

1. Use of ellagic acid, its salts, its metal complexes or its mono- or polyether or mono- or polyacylated derivatives as a cosmetic agent for improving the cohesion between the dermis and the epidermis, said agent being incorporated into a cosmetic composition comprising a cosmetically acceptable vehicle.

2. Use of ellagic acid, its salts, its metal complexes or its mono- or polyether or mono- or polyacylated derivatives as a cosmetic agent for increasing the proportion of collagen VII, by keratinocytes or fibroblasts, said agent being incorporated into a cosmetic composition comprising a cosmetically acceptable vehicle.

3. Use according to claim 1 or 2, **characterized in that** said composition is intended for toning up the skin, preventing or delaying the appearance of signs of skin ageing, and delaying the appearance of wrinkles or reducing their depth.

4. Use according to claim 3, **characterized in that** the skin ageing is actinic ageing due in particular to the action of solar radiation.

5. Use according to claim 2, **characterized in that** said composition is a hair composition for improving hair condition.

6. Use of ellagic acid, its salts, its metal complexes or its mono- or polyether or mono- or polyacylated derivatives for the preparation of a pharmaceutical composition, especially dermatological composition, for treating pathological conditions associated with a deficiency in the cohesion between the dermis and the epidermis, particularly conditions associated with a weakening of the dermal-epidermal junction.

7. Use of ellagic acid, its salts, its metal complexes or its mono- or polyether or mono- or polyacylated derivatives for the preparation of a pharmaceutical composition, especially dermatological composition, for stimulating synthesis of collagen VII by keratinocytes or fibroblasts for treating manifestations or pathological conditions associated with an insufficiency of collagen VII.

8. Use according to claim 6, **characterized in that** said composition is intended for treating epidermolysis bullosa.

9. Use according to one of claims 1 to 8, **characterized in that** the ellagic acid salts include metal salts, particularly those of alkali metals or alkaline earth metals such as sodium and calcium, amine salts such as those of methylglutamine, diethanolamine, triethanolamine, choline and bis-triethylamine, and amino acid salts, especially those of basic amino acids such as arginine, lysine and ornithine, the metal complexes of ellagic acid include those with zinc and copper, the mono- or polyacylated derivatives comprise saturated or unsaturated acyl groups having from 2 to 22 carbon atoms, these acyl groups preferably corresponding to acetic, palmitic, oleic, linoleic, linolenic, arachidonic, stearic, brassidic, erucic, behenic and (all Z)-5,8,11,14,17-eicosapentaenoic acids, and the above-mentioned mono- or polyether derivatives are derivatives of alkoxy comprising from 1 to 4 carbon atoms, or condensation derivatives of one or more of the hydroxyl groups of ellagic acid with a sugar or a chain of sugars, particularly 3-methoxyellagic acid or its mono- or polyether derivatives with sugars such as glucose, arabinose, rhamnose and galactose.

10. Use according to one of claims 1 to 9, **characterized in that** said composition contains from 0.0001% to 5% by weight, preferably between 0.01% and 1% by weight, of ellagic acid, its salts, its metal complexes or its mono- or polyether or mono- or polyacylated derivatives, based on the total weight of the composition.

11. Use according to one of claims 1 to 10, **characterized in that** said composition also contains at least one substance which favors the synthesis of the constituents of the extracellular matrix of the skin and/or regulates the formation of a good-quality corneal layer.

12. Use according to claim 11, **characterized in that** said substance is selected from the group consisting of vitamins, particularly vitamins of groups A and C, and their derivatives such as esters, tocopherols, xanthines, particularly caffeine or theophylline, retinoids, particularly vitamin A acid, extracts of Centella asiatica, asiatic and madecassic acids and their glycosylated derivatives such as asiaticoside or madecassoside, extracts of Siegesbeckia orientalis, extracts of Commiphora mukul and extracts of Eriobotrya japonica, and mineral elements.

13. Use according to claim 12, **characterized in that** the above-mentioned mineral elements are selected from magnesium, manganese, silicon and zinc, these mineral elements advantageously being used in the form of the chloride

in the case of magnesium and manganese, in the form of silanol in the case of silicon and in the form of aspartate in the case of magnesium.

**14.** Use according to one of claims 1 to 13, **characterized in that** said composition also contains at least one substance selected from the group consisting of aliphatic $C_3$-$C_{12}$ alpha-hydroxy acids, particularly citric acid, malic acid and lactic acid, amino acids, particularly arginine, citrulline and threonine, ceramides, glycoceramides, phospholipids, slimming agents, particularly forskolin, extracts of Coleus, extracts of Tephrosia, agents for combating stretch marks, particularly extracts of horse-chestnut and escin, agents for protecting or improving the microcirculation, particularly the bioflavonoids of Ginkgo biloba, and sun filters, particularly titanium oxides, acyl methoxycinnamate and filters of vegetable origin.

**15.** Use according to one of claims 1 to 14, **characterized in that** said composition also contains at least one other active principle selected from the group consisting of antidandruff agents such as extracts of Arctium lappa, chloroxylenol, resorcinol or zinc pyrithione, antiseborrhea agents such as a 5α-reductase inhibitor, particularly an extract of Pygeum africanum, and agents for stimulating the blood microcirculation, such as cepharanthine and methyl nicotinate.

**16.** Method of cosmetic treatment for improving the cohesion between the dermis and the epidermis, particularly by reinforcing the dermal-epidermal junction, and for stimulating the synthesis of collagen VII by keratinocytes or fibroblasts, **characterized in that** it consists in delivering a cosmetically effective amount of ellagic acid, its salts, its metal complexes or its mono- or polyether or mono- or polyacylated derivatives, optionally contained in a composition comprising a cosmetically acceptable excipient, particularly in a form as defined in one of claims 9 to 15.

## Patentansprüche

**1.** Kosmetische Verwendung von Ellagsäure, deren Salzen, deren Metallkomplexen, deren Mono- oder Polyetherderivaten, mono- oder polyacylierten Derivaten, als kosmetisches Mittel zur Verbesserung des Zusammenhalts zwischen der Dermis und der Epidermis, wobei das Mittel in einer kosmetischen Zusammensetzung enthalten ist, die einen kosmetisch akzeptablen Träger umfasst.

**2.** Kosmetische Verwendung von Ellagsäure, deren Salzen, deren Metallkomplexen, deren Mono- oder Polyetherderivaten, mono- oder polyacylierten Derivaten, als kosmetisches Mittel, das darauf ausgerichtet ist, die Kollagen VII-Synthese durch Keratinozyten oder Fibroblasten anzuregen, wobei das Mittel in einer kosmetischen Zusammensetzung enthalten ist, die einen kosmetisch akzeptablen Träger umfasst.

**3.** Verwendung gemäß Anspruch 1 oder 2, **dadurch gekennzeichnet, dass** die Zusammensetzung darauf ausgerichtet ist, eine Straffung der Haut zu erreichen, das Auftreten von Zeichen der Hautalterung zu verhindern oder zu verzögern, das Auftreten von Falten zu verzögern oder ihre Tiefe zu verringern.

**4.** Verwendung gemäß Anspruch 3, **dadurch gekennzeichnet, dass** die Hautalterung eine heliogene Alterung ist, insbesondere aufgrund der Wirkung von Sonnenstrahlen.

**5.** Verwendung gemäß Anspruch 2, **dadurch gekennzeichnet, dass** die Zusammensetzung eine kapillare Zusammensetzung ist, die darauf ausgerichtet ist, den Zustand der Kopfbehaarung zu verbessern.

**6.** Verwendung von Ellagsäure, deren Salzen, deren Metallkomplexen, deren Mono- oder Polyetherderivaten, mono- oder polyacylierten Derivaten, zur Herstellung einer pharmazeutischen Zusammensetzung, insbesondere einer dermatologischen, die darauf ausgerichtet ist, Erkrankungen, die mit einem Mangel des Zusammenhalts zwischen der Dermis und der Epidermis zusammenhängen, insbesondere solche, die mit einer Schwächung der dermal-epidermalen Verbindung zusammenhängen.

**7.** Verwendung von Ellagsäure, deren Salzen, deren Metallkomplexen, deren mono- oder polyacylierten Polyetherderivaten zur Herstellung einer pharmazeutischen Zusammensetzung, insbesondere einer dermatologischen, die darauf ausgerichtet ist, die Synthese von Kollagen VII durch Keratinozyten oder Fibroblasten zu stimulieren, um Manifestierungen oder Erkrankungen, die mit einem Kollagen VII-Mangel zusammenhängen, zu behandeln.

**8.** Verwendung gemäß Anspruch 6, **dadurch gekennzeichnet, dass** die Zusammensetzung auf die Behandlung

von Epidermolysis bullosa ausgerichtet ist.

**9.** Verwendung gemäß einem der Ansprüche 1 bis 8, **dadurch gekennzeichnet, dass** die Ellagsäuresalze ein Metallsalz, insbesondere ein Alkali oder Erdalkali, wie Natrium oder Calcium, Aminsalze, wie Methylglutamin-, Diethanolamin-, Triethanolamin-, Cholin-, Bis-triethylaminsalz, die Aminosäuresalze, insbesondere basische Aminosäuresalze, wie Arginin, Lysin und Ornithin, umfassen, wobei die Metallkomplexe der Ellagsäure einen Metallkomplex mit Zink und Kupfer umfassen, und die mono- oder polyacylierten Derivate Acylgruppen umfassen, die gesättigt oder ungesättigt sind, mit 2 bis 22 Kohlenstoffatomen, bevorzugt Acylgruppen, die der Essigsäure, Palmitinsäure, Oleinsäure, Linolsäure, Linolensäure, Arachidonsäure, Stearinsäure, Brassidinsäure, Erucasäure, Behensäure und (alle Z)-5,8,11,14,17-Eicosapentansäuren entsprechen; die oben erwähnten Mono- oder Polyetherderivate sind Alkoxyderivate, die 1 bis 4 Kohlenstoffatome umfassen, oder aber Kondensationsderivate von einer oder mehreren Hydroxygruppen der Ellagsäure mit einem Zucker oder einer Zuckerkette, insbesondere 3-Methoxyellagsäure oder deren Mono- oder Polyetherderivate mit Zuckern wie Glukose, Arabinose, Rhamnose und Galaktose.

**10.** Verwendung gemäß einem der Ansprüche 1 bis 9, **dadurch gekennzeichnet, dass** die Zusammensetzung, bezogen auf das Gesamtgewicht der Zusammensetzung, 0,0001 bis 5 und bevorzugt zwischen 0,01 und 1 Gew.-% Ellagsäure, an deren Salze, an deren Metallkomplexen, an deren Monooder Polyetherderivaten und mono- oder polyacylierten Derivaten enthält.

**11.** Verwendung gemäß einem der Ansprüche 1 bis 10, **dadurch gekennzeichnet, dass** die Zusammensetzung unter anderem mindestens eine Substanz enthält, die die Synthese von Bestandteilen der extrazellulären Matrix der Haut und/oder die Bildung einer Hornhautschicht von guter Qualität fördert.

**12.** Verwendung gemäß Anspruch 11, **dadurch gekennzeichnet, dass** die Substanz aus der Gruppe ausgewählt wird, die aus Vitaminen, insbesondere den Vitaminen der Gruppen A und C und deren Derivaten, wie beispielsweise den Estern, den Tocopherolen, den Xanthinen, insbesondere dem Koffein oder dem Theophyllin, den Retinoiden, insbesondere der Vitamin A-Säure, den Extrakten von Centella asiatica, den asiatischen Säuren, den Madekassische Säuren und deren glykosylierten Derivaten, wie dem Asiatikosid oder Madekassoid, den Extrakten von Siegesbeckia orientalis, den Extrakten von Commiphora mukul und den Extrakten von Eriobotyra japonica und mineralischen Elementen besteht, ausgewählt wird.

**13.** Verwendung gemäß Anspruch 12, **dadurch gekennzeichnet, dass** die oben erwähnten mineralischen Elemente aus Magnesium, Mangan, Silizium und Zink ausgewählt sind, wobei diese mineralischen Elemente vorzugsweise in Form eines Chlorids beim Magnesium und Mangan, in Form von Silanol beim Silizium und in Form von Aspartat beim Magnesium verwendet werden.

**14.** Verwendung gemäß einem der Ansprüche 1 bis 13, dadurch charakterisiert, dass die Zusammensetzung unter anderem mindestens eine Substanz enthält, wird aus der Gruppe, die aus $C_3$-$C_{12}$ aliphatischen Alpha-Hydroxysäuren, insbesondere Zitronensäure, Maleinsäure und Milchsäure, den Aminosäuren, insbesondere Arginin, Citrullin und Threonin, den Ceramiden, den Glykoceramiden, den Phospholipiden, den Verflüssigungsmitteln, insbesondere Forskolin, den Buntnesselextrakten, den Tephrosia-Extrakten, den Mitteln gegen Artrophia striata et marculata, insbesondere Extrakten aus Marron d'Inde und Escin, den Stoffen zur Förderung und Verbesserung der Mikrozirkulation, insbesondere Bioflavonoiden aus Gingko Biloba und den Sonnenfiltern, insbesondere Titanoxiden, Methoxyacylcinnamat und Filtern pflanzlichen Ursprungs gebildet wird, ausgewählt.

**15.** Verwendung gemäß einem der Ansprüche 1 bis 14, **dadurch gekennzeichnet, dass** die Zusammensetzung unter anderem mindestens einen anderen aktiven Wirkstoff enthält, der aus der Gruppe, die aus den Antischuppen-Mitteln, wie beispielsweise Extrakten von Arctium lappa, Chlorxylenol, Resorcin oder Zinkpyrithion, den antiseborrhoischen Mitteln, wie beispielsweise einem 5α-Reduktaseinhibitor, insbesondere dem Extrakt aus Pygeum africanum, und den Mitteln, die die Mikrozirkulation des Bluts stimulieren, wie beispielsweise Cepharanthin und Methylnikotinat gebildet wird, ausgewählt wird.

**16.** Kosmetisches Behandlungsverfahren, das darauf ausgerichtet ist, eine Verbesserung des Zusammenhalts zwischen der Dermis und Epidermis zu erhalten, insbesondere durch Verstärkung der dermal-epidermalen Verbindung und/oder zum Stimulieren der Synthese von Kollagen VII durch Keratinozyten oder Fibroblasten, **dadurch gekennzeichnet, dass** es daraus besteht, eine wirksame kosmetische Menge Ellagsäure, an deren Salzen, an deren Metallkomplexen, an deren Mono- oder Polyetherderivaten und mono- oder polyacylierten Derivaten, die

gegebenenfalls in einer Zusammensetzung enthalten sind, die einen kosmetisch akzeptablen Trägerstoff umfasst, insbesondere in einer derartigen Form, wie in den Ansprüchen 9 bis 15 definiert, zu verabreichen.